# EUROPÄISCHE PATENTSCHRIFT

(11) **EP 1 395 679 B1**
(45) Veröffentlichungstag und Bekanntmachung des Hinweises auf die Patenterteilung: **07.07.2010**
(21) Anmeldenummer: 02726163.5
(22) Anmeldetag: 13.03.2002
(51) Int. Cl.: C12Q 1/68, B01J 19/00

(54) **NUKLEOTIDTRÄGER ZUR DIAGNOSE UND THERAPIE ORALER ERKRANKUNGEN**
NUCLEOTIDE CARRIER FOR DIAGNOSING AND TREATING ORAL DISEASES
SUPPORT NUCLEOTIDIQUE DESTINE AU DIAGNOSTIC ET A LA THERAPIE DE MALADIES BUCCALES

(30) Priorität: 13.03.2001 DE 10112348
(43) Veröffentlichungstag der Anmeldung: 10.03.2004
(73) Patentinhaber: Carpegen GmbH, 48149 Münster (DE)
(72) Erfinder: RÖTGER, Antje, 48147 Münster (DE)
(74) Vertreter: König, Gregor Sebastian
(86) Internationale Anmeldenummer: PCT/EP2002/002781
(87) Internationale Veröffentlichungsnummer: WO 2002/072883

(56) Entgegenhaltungen:
- EP-A- 0 404 210
- EP-A- 0 769 560
- US-A- 5 378 604
- US-A- 5 789 174
- SLOTS C.A. ET AL.,: "typing of herpes simplex virus from human periodontium" ORAL MICROBIOL. IMMUNOL., Bd. 16, Februar 2001 (2001-02), Seiten 63-64, XP002239246
- SAKAMOTO M ET AL: "COMPARISON OF THE ORAL BACTERIAL FLORA IN SALIVA FROM A HEALTHY SUBJECT AND TWO PERIODONTITIS PATIENTS BY SEQUENCE ANALYSIS OF 16S RDNA LIBRARIES" MICROBIOLOGY AND IMMUNOLOGY, TOKYO, JP, Bd. 44, Nr. 8, 2000, Seiten 643-652, XP001079903 ISSN: 0385-5600
- SCHENA M ET AL: "PARALLEL HUMAN GENOME ANALYSIS: MICROARRAY-BASED EXPRESSION MONITORING OF 1000 GENES" PROCEEDINGS OF THE NATIONAL ACADEMY OF SCIENCES OF USA, NATIONAL ACADEMY OF SCIENCE. WASHINGTON, US, Bd. 93, Nr. 20, 1. Oktober 1996 (1996-10-01), Seiten 10614-10619, XP002022507 ISSN: 0027-8424

## Beschreibung

Die Erfindung betrifft einen Nukleotidträger zur Diagnose und Therapie oraler Erkrankungen, insbesondere der Parodontitis und nimmt die Priorität der deutschen Patentanmeldung 101 12 348.5 in Anspruch, auf die inhaltlich Bezug genommen wird.

Die Parodontitis ist eine entzündliche Erkrankung des Zahnbettes, die unbehandelt zu einem Gewebe- und Knochenabbau des Zahnhalteapparats und damit letztlich zu einem Verlust der Zähne führt. Sie gehört zu einem der häufigsten Krankheitsbilder unserer Gesellschaft, von der ca. 35 bis 40% aller erwachsenen Deutschen betroffen sind. Die Parodontitis ist eine multifaktorielle Erkrankung, die durch eine Vielzahl verschiedener Mikroorganismen ausgelöst wird und deren Verlauf durch individuelle Verhaltensweisen - z.B. die Mundhygiene oder starkes Rauchen - und bestimmte Stoffwechselerkrankungen wie beispielsweise die Diabetes sowie eine genetische Dispositionen beeinflußt wird.

Der primäre Mechanismus der Parodontitis liegt in der Bildung bakterieller Plaque, die sich am Zahnfleischsaum zu Zahnstein verhärtet und zu einer mechanischen Reizung führt. Die Virulenzfaktoren der Plaquebakterien verursachen im angrenzenden Zahnfleisch Rötungen und Schwellungen, die das klinische Erscheinungsbild der Zahnfleischentzündung (Gingivitis) erzeugen. Die Gingivitis kann sich zu einer Parodontitis fortentwickeln. Dies geschieht entweder direkt, indem bakterielle Toxine, Enzyme oder Stoffwechselprodukte - die zu den Virulenzfaktoren zählen - das umgebende Gewebe schädigen, oder indirekt, indem diese Faktoren Entzündungsmechanismen des Immunsystems aktivieren, die ihrerseits zur Zerstörung des Knochen- und Weichgewebes des Zahnhalteapparats beitragen. Mit Fortschreiten der Erkrankung weicht das Zahnfleisch zurück - Verlust des sog. *atfachments -* so daß sich tiefe Parodontaltaschen (sog. Sulkus) bilden, die ein ideales Ökosystem für anaerobe Bakterien darstellen. Bei der Entstehung einer Parodontitis verschiebt sich daher das Spektrum der Bakterien von aeroben grampositiven Kokken und Stäbchen zu anaeroben gramnegativen Stäbchen.

Die Gesamtheit der parodontalen (oder oralen) Erkrankungen läßt sich in die *Gingivitis* (Zahnfleischentzündung), die *chronische adulte Parodontitis (AP),* die *refraktäre Parodontitis,* die *Periimplantitis* und die akuten oder rasch progressiven Parodontopathien unterteilen. Zu der letzteren Gruppe gehört die *ANUG (akute nekrotisierende-ulzerierende Gingivitis),* die *EOP ("early-onset periodontitis"),* die *RPP (rasch verlaufende, progressive Parodontitis)* und die *LJP (Lokalisierte juvenile Parodontitis).*

Die unterschiedlichen Krankheitsbilder gehen jeweils mit einem charakteristischen Keimspektrum - d.h. mit einem quantitativ und qualitativ bestimmt zusammengesetzten Spektrum an Bakterien - einher. So zeichnen sich die Gingivitis und die chronische adulte Parodontitis durch ein in Richtung gramnegativer, anaerober Bakterien verschobenes Keimspektrum aus. Die RPP und die refraktäre Parodontitis weisen oft hohe Keimzahlen Parodontitis-assoziierter Bakterien auf. Das Keimspektrum der LJP ist hingegen durch den Leitkeim *Actinobacillus actinomycetemcomitans* bestimmt. Nach der sog. spezifischen Plaquehypothese ist es in der Fachwelt anerkannt, daß der Schweregrad einer Parodontitis in deutlicher Korrelation zum Vorkommen bestimmter Bakterienspezies steht. Diese parodontalpathogenen Markerkeime sind anaerob oder mikroaerophil, so dass sie in den tiefen Teilen des Sulkus ihr pathogenes Potential entfalten können.

Die Pathogenität dieser Bakterien liegt in ihren Virulenzfaktoren begründet, die beispielsweise als Adhäsine (u.a. Haftpili, Fimbrien) und Oberflächenkohlenhydrate die Anhaftungsfähigkeit, sowie als Leukotoxine, Kapseln oder Chemotaxis-Inhibitoren die Fähigkeit zur Abwehr des Wirtsimmunsystems oder zum Verdrängen der benignen Parodontalflora oder als Proteasen, Epitheliotoxine oder Endotoxine die Gewebsinvasivität der Keime vermitteln. Die Virulenzfaktoren sind außerdem für systemische Wirkungen der Bakterien verantwortlich, wobei die Virulenz, d.h. das Ausmaß der krankheitserzeugenden Eigenschaft eines Stammes einer pathogenen Spezies, bei verschiedenen Stämmen sehr unterschiedlich sein kann.

Die spezifische Plaquehypothese ist jedoch nicht geeignet, die Entstehung der Parodontitis eindeutig zu beschreiben. So konnten dieselben Parodontitis-assoziierten Keime z. B. auch in vielen parodontal gesunden Personen nachgewiesen werden. Zur Krankheitsentstehung muss daher u.a. auch eine individuelle Anfälligkeit des Patienten hinzukommen. Diese kann einerseits ihre Ursache in einer genetischen Disposition haben, andererseits aber auch durch die Immunabwehr schädigende Umweltfaktoren und Allgemeinerkrankungen vermittelt werden (z. B. Rauchen, psychischer Stress, Diabetes mellitus).

Neben diesen plaque-spezifischen Bakterien sind - so wird vermutet - für die Ausbildung oraler Erkrankungen auch Mikroorganismen relevant, die in der subgingivalen Plaque normalerweise nicht vorkommen, bei Patienten aber, die schlecht auf eine Parodontaltherapie ansprechen, häufig nachzuweisen sind. Vor allem Darmbakterien, Staphylococcen und andere untypische Mundbewohner konnten in einer Studie bei 14% der Patienten mit adulter Parodontitis nachgewiesen werden, davon die Arten *Enterobacter cloacae, Klebsiella pneumoniae, Pseudomonas aeruginosa, Klebsiella oxytoca* und *Enterobacter agglomerans* in über 50% der Fälle. Auch opportunistische Pilze/Hefen (z. B. *Candida* spec.) konnten bei Parodontitispatienten mit immunsupprimierenden Faktoren, wie z. B. Rauchen, nachgewiesen werden.

Auch Viren können zu den Parodontitis-assoziierten Mikroorganismen gehören. Orale Erkrankungen konnten beispielsweise inzwischen mit einer Reihe von Herpesviren in Zusammenhang gebracht werden, darunter Herpes Simplex Virus 1 und 2 (HSV-1 und HSV-2), Varizella-Zoster-Virus (VZV), Epstein-Barr Virus (EBV), Zytomegalievirus (HCMV) und Humanes Herpes Virus 8 (HHV-8).

Einen entscheidenden Einfluss auf die Progression der Parodontitis hat das Immunsystem des Patienten. Dies kann zwar die Vermehrung eindringender Bakterien verhindern helfen, trägt selber aber auch zu einer verstärkten Zerstörung des Gewebes bei, indem die Botenmoleküle der Immunzellen auch destruktiv auf das eigene Gewebe wirken, da z. B. aktivierte Makrophagen Osteoklasten (Knochensubstanz abbauende Zellen) aktivieren und damit zum Abbau des Alveolarknochens (Kieferknochens) beitragen.

Entzündungsfördernde Botenstoffe (Zytokine) werden in Abhängigkeit von der genetischen Disposition von verschiedenen Patienten in unterschiedlichem Ausmaß gebildet. Relevante genetische Unterschiede konnten inzwischen für die Produktion der Zytokine Interleukin-1A (IL-1A) und Interleukin-1 B (IL-1B), des Interleukin-1 Rezeptor Antagonisten (IL-1 RA), des Interleukin-4 (IL-4), des Tumor-Nekrose-Faktors α (TNFα), des Fcγ RII (CD32)-Rezeptors und für die HLA-Antigene HLA-A9 und HLA-B15 nachgewiesen werden [Meyle J, Domann E, Gonzales J (2000). Molekularbiologische PAR-Diagnostik und ihre Bedeutung. Workshop 5 auf der Jahrestagung der Deutschen Gesellschaft für Parodontologie, Frankfurt, 2000.].

Bei vielen Patienten mit Gingivitis oder leichter bis mittelschwerer Parodontitis lässt sich das Fortschreiten der Erkrankung durch konventionelle Behandlungsmethoden (Mundhygieneinstruktion, mechanische Verfahren zur Entfernung von supra- und subgingivaler Plaque, evtl. Lappenoperation) verhindern. Daneben tritt bei einigen Patienten eine unterstützende antibiotische Therapie. Bei Vorhandensein von *A. actinomycetemcomitans* oder z. B. von *P. gingivalis* und *B. forsythus* in hoher Keimzahl kann die Antibiotikatherapie notwendig sein, wobei aber auch die individuelle immunologische Konstitution des Patienten den Verlauf und damit die Notwendigkeit der Antibiotikatherapie bestimmt.

Die aktuellen Antibiotikaempfehlungen für eine Therapie richten sich nach dem Keimspektrum des Patienten. Die gegenwärtig möglichen Empfehlungen beschränken sich allerdings darauf, bei Vorhandensein von *A. actinomycetemcomitans* eine andere Antibiotikatherapie zu wählen als bei der Abwesenheit dieses Keims.

Die Pathogenese oraler Erkrankungen, insbesondere der Parodontitis, ist ein komplexer Prozeß, der im wesentlichen von dem Spektrum der Parodontitis direkt oder indirekt assoziierten Mikroorganismen, den jeweiligen relevanten Virulenzfaktoren und der genetischen Disposition des Betroffenen bestimmt wird. Die vorhandenen Mikroorganismen - mit ihren etwaigen Antibiotikaresistenzen - sind ihrerseits maßgeblich für den angestrebten Therapieerfolg. Eine möglichst genaue Diagnose und eine die Diagnoseergebnisse berücksichtigende Therapie macht daher zunächst die individuelle Bestimmung dieser Einflußgrößen bei dem Betroffenen erforderlich.

Dazu ist es bekannt, den Nachweis und die Identifizierung pathogener Bakterien, Pilze bzw. Viren über mikroskopische Methoden oder die Kultivierung dieser Mikroorganismen auf Selektionsmedien zu führen. Diese Verfahren sind jedoch nicht nur wegen der Kultivierungsdauer und ihrer geringen Sensitivität nachteilig, sondern sind insbesondere für anaerobe Mikroorganismen ungeeignet. Auch sind viele Parodontitis assoziierte Mikroorganismen nicht *in vitro* kultivierbar. Alternativ können Mikroorganismen über Immunoassays nachgewiesen werden. Dieser Ansatz ist aber ebenso durch die geringe Sensitivität und zudem durch die begrenzte Verfügbarkeit und Spezifität der notwendigen Antiseren beschränkt.

Daneben ist u.a. eine Identifizierung der Mikroorganismen über den direkten Nachweis ihrer DNA oder RNA möglich, der auf dem Einsatz bekannter Hybridisierungsverfahren wie Southern- oder Northern-Blot-Analysen basiert. Mit diesen Tests können jedoch meist aus Kosten- und Zeitgründen nur 3 bis 5 Arten gleichzeitig nachgewiesen werden können [Dix K, Watanabe SM, McArdle S, Lee DI, Randolph C, Moncla B, Schwartz DE (1990). Speciesspecific oligonucleotide probes for the identification of periodontal bacteria. J Clin Microbiol, 28: 319-323].

Ähnliche Schwierigkeiten herrschen beim Austesten mikrobieller Antibiotika-Resistenzen, die entweder auf beispielsweise durch Plasmidtransfer erworbene Resistenzgene oder auf Mutationen bakterieneigener Gene zurückgehen können (beide genetischen Ursachen werden nachfolgend als "Antibiotika-Resistenzgene" bezeichnet). Dazu werden die Mikroorganismen üblicherweise in Gegenwart antibiotisch wirksamer Substanzen kultiviert, so daß die Kultivierungsdauer sowie die Exposition der Kulturen an Sauerstoff die bereits geschilderten Probleme bereiten.

Die genetische Disposition des Menschen zur Ausbildung einer oralen Erkrankung wird gegenwärtig nur über ein Screening von zwei Genpolymorphismen des Interleukin 1 (IL-1A und IL-1B) berücksichtigt.

Die bekannten Verfahren zur Diagnose oraler Erkrankungen haben den Nachteil, daß keines für sich genommen geeignet ist, der Komplexität der Pathogenese - nämlich der Bedeutung der quantitativen und qualitativen Zusammensetzung der oralen Mikroorganismen, ihrer Virulenzfaktoren sowie der genetischen Disposition des Patienten - gerecht zu werden. Ebensowenig ermöglichen diese bekannten Tests eine Aussage über etwaige antibiotische Therapieansätze.

Der Erfindung liegt daher die Aufgabe zugrunde, die Diagnose und die Therapie oraler und parodontaler Erkrankungen, insbesondere der Parodontitis zu verbessern.

Diese Aufgabe wird gelöst durch einen Nukleotidträger nach einem der unabhängigen Ansprüche. Vorteilhafte Weiterentwicklungen sind Gegenstand entsprechender Unteransprüche.

Der Erfindung liegt dabei der Gedanke zugrunde, einen Träger mit Sonden für Nukleotid-Referenzsequenzen (nachfolgend Referenzsequenzen) zu versehen, die entweder für die genetische Disposition des Patienten für orale Erkrankungen, für die Identifizierung eines Parodontitis assoziierten Mikroorganismus, für die Ermittlung der mikrobiellen Virulenzfaktoren oder der Ausbildung mikrobieller Antibiotikaresistenzen spezifisch sind. Diese Sonden, die in definierter Anordnung in vielfachen Serien auf den Träger (nachfolgend auch Genchip) aufgebracht werden können, dienen zum Nachweis der aus Patientenproben amplifizierten Nukleotidsequenzen. Diese können entweder mikrobiellen oder humanen Ursprungs sein. Die Referenzsequenzen können während der Amplifizierung beispielsweise mit Fluoreszenzfarbstoffen markiert werden, so daß ihre etwaige anschließende Hybridisierung mit den auf dem Chip aufgebrachten Sonden qualitativ oder quantitativ nachweisbar ist. Alternativ können die Sonden auch die zu den Referenzsequenzen komplementäre Nukleotidsequenzen (nachfolgend auch Komplementärsequenzen) erkennen.

Vorteilhafterweise werden auf den Träger Sonden sowohl für die Untersuchung der humanen Parodontitis assoziierten Genpolymorphismen als auch für die Analyse des Erregerspektrums sowie für die mikrobiellen Antibiotikaresistenzen und Virulenzfaktoren aufgebracht. Damit erlaubt der erfindungsgemäße Genchip die simultane Ermittlung dieser Parameter und ermöglicht so die einfache und schnelle Erstellung eines individuellen "Parodontitisprofils" für den jeweiligen Patienten zur besseren Prognose und Therapie dieser multifaktoriell bedingten Erkrankung.

Bei den Referenzsequenzen handelt es sich vorzugsweise um Nukleotidsequenzen, die entweder für das den Untersuchungsparameter codierende Gen bzw. den Genabschnitt oder den Erreger oder aber für Mutationen davon spezifisch sind. Entsprechendes gilt für die Komplementärsequenzen. Soll z.B. der erfindungsgemäße Chip der Untersuchung des Erregerspektrums dienen, können komplementäre Sequenzen der jeweiligen erregerspezifischen variablen Region des Gens für 16SrRNA als Sonde auf den Chip aufgebracht werden. Soll dagegen beispielsweise eine Analyse bakterieller Resistenzgene erfolgen, können spezifische Oligonukleotidsequenzen aus den Resistenzgenen (oder dazu komplementäre Sequenzen) als Sonden verwendet werden. Ebenso können homologe Nukleotidsequenzen oder allelische Varianten davon als Sonden dienen.

Die als Sonden auf den Chip aufgetragenen Referenzsequenzen bzw. die Komplementärsequenzen können unterschiedlich lang sein. Vorzugsweise werden jedoch 16- bis 25-mere verwendet. Insbesondere eignen sich 15-mere.

In einer besonders vorteilhaften Ausführungsform kann der Chip nicht nur der qualitativen Bestimmung sondern ebenso der Quantifizierung einzelner Erreger in der Untersuchungsprobe dienen. Dazu können die Sonden in einem großen Überschuß gegenüber der in der Patientenprobe zu erwartenden und amplifizierten Anzahl der Erreger aufgebracht werden. Nach erfolgter Hybridisierung auf dem Chip kann diese in einem Scanner quantitativ ausgewertet werden.

Vorzugsweise werden mit dem Chip die in Tabelle 9 aufgeführten Mikroorganismen untersucht. Damit werden einerseits Erreger erfaßt, deren Zusammenhang mit der Parodontitis nachgewiesen ist. Daneben werden jedoch auch orale Bakterien erfaßt, deren Beteiligung nur vermutet oder noch unklar ist. Darüber werden in dieser Ausführungsform auch Mikroorganismen nachgewiesen, die ebenso in der gesunden oralen Flora vorkommen oder an Karies beteiligt sind. Zusammenfassend werden alle diese Mikroorganismen als "Parodontitis assoziiert" bezeichnet. Damit wird ein quantitativer und qualitativer Überblick über das gesamte Erregerspektrum möglich, der eine Voraussetzung für die aussagekräftige Einschätzung des Stadiums der parodontalen Erkrankung sowie der Karies erlaubt. Dabei erfolgt auch der simultane Nachweis von allen bekannten humanen Herpesviren sowie von opportunistischen (fakultativ pathogenen) Erregern wie z. B. den Pilzorganismen *Candida albicans* und *Aspergillus fumigatus* bzw. Staphylococcen, Streptococcen, Pseudomonaden, etc.

Der Genchip ermöglicht - wie bereits ausgeführt - die Ermittlung des individuellen Parodontitis-Profils eines Patienten. Die Kenntnis des Parodontitis-Profils ist jedoch nicht nur für die Verbesserung der Prognose und Therapie dieser Erkrankung hilfreich, sondern erbringt darüber hinaus auch Hinweise auf die Pathogenese von nach derzeitigem Kenntnisstand mit der Parodontitis assoziierten Erkrankungen. So wird beispielsweise ein Zusammenhang zwischen der Parodontitis und einem erhöhten Risiko für koronare Herzkrankheit vermutet, da die Bakterien nach neueren Untersuchungen offenbar in der Lage sind, einerseits mittels der durch den Bakterienbefall ausgelösten Zytokinproduktion das Gerinnungssystem spezifisch zu beeinflussen und so eine Atherogenese zu initiieren. Andererseits können auch die bakteriellen Oberflächenmoleküle und Proteasen oder auch die Bakterien selbst die Thrombozytenaggregation und -aktivierung induzieren [Offenbacher S, Madianos PN, Champagne CM, Southerland JH, Paquette DW, Williams RC, Slade G, Beck JD (1999). Periodontitis-atherosclerosis syndrome: an expanded model of pathogenesis. J Periodontal Res, 34: 346-352.].

Auch ist es bekannt, daß eine schwere Parodontitis die Ausprägung der Symptome bei einem Diabetes-Patienten verstärken kann, indem den Kohlenhydratstoffwechsel beeinflussende Zytokine infektionsbedingt hochreguliert werden [Grossi SG, Genco RJ (1998). Periodontal disease and diabetes mellitus: a two-way relationship. Ann Periodontol, 3: 51-61.]. Darüber hinaus können orale Infektionen, insbesondere Parodontitis, ursächlich mit bakteriellen Pneumonien, Frühgeburten und einem niedrigen Geburtsgewicht zusammenhängen [Engebretson SP, Lalla E, Lamster IB (1999). Periodontitis and systemic disease. N Y State Dent J, 65: 30-32; Li X, Kolltveit KM, Tronstad L, Olsen I (2000). Systemic diseases caused by oral infections. Clin Microbiol Rev, 13: 547-558.]. Auch hier kann demnach der erfindungsgemäße Chip zur Verbesserung der Diagnose der Erkrankungen und anschließender Therapie beitragen.

Sofern der Chip auch Sonden für Referenzsequenzen für Karieserreger (beispielsweise *Actinomyces* spec., *Lactobacillus* spec., *Streptococcus* spec. und *Candida* spec.) aufweist, kann er ebenso zur Kariesdiagnose verwendet werden, da das individuelle Kariesrisiko von der Art und Anzahl dieser Erreger abhängig ist [Kayser FH: Erreger bakterieller Infektionskrankheiten. In: Kayser FH, Bienz KA, Eckert J, Lindenmann J (Hrsg.) Medizinische Mikrobiologie: Immunologie, Bakteriologie, Mykologie, Virologie, Parasitologie, 1993, Begr. von E. Wiesmann, Thieme, Stuttgart, New York, p. 164; Raitio M, Pienihakkinen K, Scheinin A (1996). Multifactorial modeling for prediction of caries increment in adolescents. Acta Odontol Scand, 54: 118-121].

Ebenso kann der Chip mit Sonden für Mikroorganismus spezifischen Referenzsequenzen zum qualitativen und ggf. auch quantitativen Nachweis von anderen Infektionskrankheiten dienen. Dies gilt insbesondere für opportunistische Infekte (z. B. Harnwegsinfekte, Wundinfekte, Hautinfektionen), die von fakultativ pathogenen bakteriellen Erregern wie z. B. *S. aureus, E. coli, Enterobacter* spec. und *Klebsiella* spec. bei abwehrgeschwächten Patienten hervorgerufen werden. Das gleiche gilt auch für die Diagnostik opportunistischer Mykosen (Pilzerkrankungen), wie z. B. Kandidose, Aspergillose sowie humaner Herpesviren, die mit dem Genchip ebenso durchgeführt werden kann.

Aufgrund des ubiquitären Vorkommens der durch den Genchip nachgewiesenen bakteriellen Resistenzgene, kann der Genchip auch zur Bestimmung von Antibiotikaresistenzen bei den Erregern beliebiger Infektionskrankheiten verwendet werden, z. B. zur Erklärung der häufigen Vielfachresistenzen bei Krankenhausinfektionen.

Vorteilhafterweise kann der Chip zu Zwecken des jeweils ausgewählten Verwendungszweckes ausschließlich mit den dafür relevanten Sonden versehen sein. Soll beispielsweise mittels des Chips nur eine Analyse der Antibiotikaresistenzen erfolgen, kann aus Kostengründen der Chip nur die dafür relevanten Sonden tragen.

Neben den genannten Anwendungsmöglichkeiten in der praxisorientierten zahnmedizinischen und medizinischen Diagnostik kann der Genchip gleichzeitig ein geeignetes Mittel für die weitergehende Erforschung oraler Infektionen darstellen. Bei vielen Erregern wird die Beteiligung an der Entstehung der Parodontitis vermutet, ohne daß ihre Rolle endgültig geklärt wäre. Mit Hilfe des Genchips können kostengünstig groß angelegte Studien durchgeführt werden, bei denen eine große Anzahl von Parametern gleichzeitig an verschiedenen Patientenkollektiven untersucht werden kann.

Für den Zweck dieser Anmeldung werden die nachfolgenden Begriffe definiert als:
Der Begriff "Nukleotidsequenz" bezieht sich auf jede oligomere oder polymere Sequenz von Desoxyribonukleotiden oder Ribonukleotiden tierischen, menschlichen, mikrobiellen oder synthetischen Ursprungs. Die Nukleotide können neben Guanin, Cytosin, Thymin oder Adenin oder Uridin auch andere natürliche oder synthetische Basenanaloga enthalten. Der Begriff "Nukleotidsequenz" wird synonym mit "Nukleinsäure" oder "Oligonukleotid" verwendet.

Der Begriff "Nukleotidträger" bezieht sich auf alle festen oder halbfesten Träger, an die mindestens eine Nukleotidsequenz gebunden ist. Dieser Begriff ist synonym mit "Chip" oder "Genchip".

Der Begriff "Referenzsequenz" bezieht sich auf Nukleotidsequenzen mit einer mindestens hinreichenden Spezifität für bestimmte Phänotypen oder Organismen, so daß ihr Vorhanden den Nachweis dieser Phänotypen oder Organismen erlaubt.

Der Begriff "Sonde" bezieht sich auf Nukleotidsequenzen, die zum Nachweis oder zur Identifizierung von Nukleotidsequenzen (Referenzsequenzen oder Komplementärsequenzen) verwendet werden. Sie können identisch oder komplementär zu der zu suchenden Nukleotidsequenzen sein oder eine Homologie dazu aufweisen. Ebenso können sie eine allelische Variante zu der zu suchenden Sequenz darstellen.

Der Begriff "Mikroorganismus" bezieht sich sowohl auf Bakterien als auch auf Viren, Pilze oder andere selbstreplizierende Strukturen.

Der Begriff "Hybridisierung" bezieht sich auf die Anlagerung zweier komplementärer oder teilkomplementärer Nukleinsäuren aneinander. Die Hybridisierung stellt einen wesentlichen Schritt in zahlreichen molekularbiologischen Verfahren dar. Mit diesen Techniken werden einzelne Gene oder Teile davon in einer DNA-Präparation oder das Transkriptionsprodukt eines Genes (mRNA) nachgewiesen [Sambrook J, Fritsch EF, Maniatis T. Molecular cloning: A laboratory manual. 2nd ed., vol. 2, 1989, Cold Spring Harbor Laboratory press, 13.96-97; Constanzi C, Gillespie D: Fast blots: immobilization of DNA and RNA from cells. In: Guide to molecular cloning techniques. Edited by Berger SR and Kimmel AR, Academic Press Inc., San Diego: Methods in Enzymology 1987, 152: p582-87; Schena M et al.: Quantitative monitoring of gene expression patterns with a complementary DNA microarray. Science 1995, 270: pp.467-470.]

Der Begriff "Virulenzfaktor" bezieht sich auf alle Eigenschaften eines Mikroorganismus, die an der Entstehung und der Aufrechterhaltung einer Infektion beteiligt sind.

Der Begriff "Keimspektrum" oder "Erregerspektrum" bezieht sich auf die quantitative und/oder qualitative Zusammensetzung einer Population von Mikroorganismen.

Nachfolgend wird der erfindungsgemäße Nukleotidträger anhand von Ausführungsbeispielen des näheren erläutert. Die Nomenklatur der in den Tabellen in Bezug genommenen Gene bezieht sich dabei auf die GenBank des NCBI.

### Beispiel 1:

### Identifizierung und Quantifizierung Parodontitis-assoziierter bzw. oralpathogener Bakterien mit dem erfindungsgemäßen Genchip

Die komplementären Nukleinsäuresequenzen der in Tabelle 1 aufgelisteten bakteriellen Referenzsequenzen werden als Sonden auf einem geeigneten Träger, z. B. mit Gold beschichtetem Glas, aufgebracht. Dazu dienen Standardtechniken, wie sie beispielsweise aus der DE 196 12 356 A1 und US 5 837 832 A bekannt sind. Diese werden zu Zwecken der Offenbarung vollumfänglich in Bezug genommen. Dabei werden in der Regel 15-mere verwendet, es können jedoch auch 16- bis 25-mere zum Einsatz kommen.

Die Referenzsequenzen entsprechen jeweils den erregerspezifischen, variablen Regionen der Gene für die 16SrRNA (ribosomale RNA für die kleine Ribosomenuntereinheit). Die 16SrRNA-Gene (= 16SrDNA) werden ausgewählt, da durch die Anwendung universeller Primer eine PCR-Reaktion zur Erhöhung der Sensitivität der Hybridisierungsreaktion vorgeschaltet werden kann und da die entsprechenden Sequenzen in allen zu untersuchenden Bakterien bekannt sind. Zu Kontrollzwecken werden die zu den Referenzsequenzen komplementären Sonden in vielfacher Kopie auf je zwei nebeneinander liegende Felder auf den Träger aufgebracht. Für die 61 in der Tabelle 1 aufgeführten Erreger (s. Tabelle 1) werden demnach 61 spezifische Sonden verwendet werden, so dass sich insgesamt 122 Felder auf dem Chip ergeben. Dabei enthalten z.B. Feld 1.1 und Feld 1.2 zur Detektion des *Actinobacillus actinomycetemcomitans* jeweils eine Vielzahl der Sequenz CAGCGTCAGTACATC (komplementär zur Referenzsequenz GATGTACTGACGCTG; Tabelle 1, Zeile 1). Für die Belegung der Felder 2.1 und 2.2 wird die Sequenzinformation der Zeile 2 der Tabelle 1 verwendet usw.

Die Quantifizierung erfolgt durch den Einsatz der DNA bekannter Bakterienmengen auf dem Chip und die Korrelation der Bakterienzahl zu den Signalstärken bei der Chip-Auswertung.

**Tabelle 1:**

| Referenzsequenzen der 16SrRNA-Gene ausgewählter Bakterienspezies, deren komplementäre Sequenzen als Sonden auf den Genchip aufgebracht werden. | | |
|---|---|---|
| **Nr.** | **Bakterienart** | **16SrDNA-Referenzsequenz** |
| 1 | *Actinobacillus actinomycetemcomitans* | GATGTACTGACGCTG |
| 2 | *Porphyromonas gingivalis* | TCAGCGGTGAAACCT |
| 3 | *Prevotella intermedia* | ATCTACCTTGCAGCG |
| 4 | *Bacteroides forsythus* | GATAAAATTGCCGTT |
| 5 | *Treponema denticola* | CTACGAGCTCAACTC |
| 6 | *Campylobacter rectus* | GTATACAATAAGACG |
| 7 | *Eubacterium nodatum* | TGTCGTATGCCCCGC |
| 8 | *Eikenella corrodens* | TTATTTAAGCAGGAT |
| 9 | *Selenomonas noxia* | ATGTAAGGATGGGCA |
| 10 | *Fusobacterium nucleatum subspecies nucleatum* | GCAAAGCCGTGAGGT |
| 11 | *Peptostreptococcus micros* | TCATTAAGCATTTGA |
| 12 | *Prevotella nigrescens* | CCTGCGGGAGTGTTA |
| 13 | *Streptococcus intermedius* | GACTTAGTGCCGCAG |
| 14 | *Streptococcus mutans* | TAGTGTGCTCTGGAA |
| 15 | *Actinomyces viscosus* | GGTCTCTGGGCCGCT |
| 16 | *Treponema socranskii* | CAACTCCGGAGCTAT |
| 17 | *Treponema pectinovorum* | TAACCCCAGAACTGC |
| 18 | *Treponema vincentii* | TTATGTAAGCCTGGT |
| 19 | *Treponema lecithinolyticum* | CACATCTTGAATTAC |
| 20 | *Campylobacter concisus* | ATATACAATGAGAAG |
| 21 | *Campylobacter gracilis* | AGACGCAATATCGTA |
| 22 | *Campylobacter sputorum* | CAGTTAGGCTGAGCA |
| 23 | *Eubacterium brachy* | ATTGTTCGCCCTATG |
| 24 | *Eubacterium timidum* | CTCTACATGCCTTGC |
| 25 | *Eubacterium lentum* | GAGGGAATCCCTCAA |
| 26 | *Pseudoramibacter alactolyticus* | TTGCTGGGCAGATAC |
| 27 | *Selenomonas sputigena* | CAAACCATCCCCCAG |
| 28 | *Fusobacterium periodonticum* | GAAACTGCATAACTA |
| 29 | *Peptostreptococcus anaerobius* | TCAACCGTAGTTAGC |
| 30 | *Prevotella melaninogenica* | TGACGGGAGCGCAAC |
| 31 | *Wolinella succinogenes* | GTTGAACTGCATTTG |
| 32 | *Veillonella parvula* | CGGATCAGTCAGTCT |
| 33 | *Veillonella dispar* | ACTGCCAATCTAGAG |
| 34 | *Centipeda periodontii* | GACTGAAGTTACTAG |
| 35 | *Dialister pneumosintes* | TCGCAATCCATAGAA |
| 36 | *Porphyromonas endodontalis* | TAGGTGGCGTATTAA |
| 37 | *Streptococcus salivarius* | AAAGTTACTTCGGTA |
| 38 | *Streptococcus sobrinus* | TCAACCAATGTATGC |
| 39 | *Streptococcus sanguis* | TTAACCATAGTATGC |
| 40 | *Lactobacillus casei* | GAGGAAGCGCATCGG |
| 41 | *Lactobacillus (Olsenella) uli* | CCCGCCCGCTCCCGA |
| 42 | *Lactobacillus (Atopobium) rimae* | TCCGCCCGCTCCCGA |
| 43 | *Actinomyces naeslundii* | TGCCGGTCTGCTCCG |
| 44 | *Actinomyces odontolyticus* | CACGGCGGCACTGCA |
| 45 | *Actinomyces israelii* | GCCTCCCTTTTTGGG |
| 46 | *Capnocytophaga gingivalis* | GGGGACTAACAGACA |
| 47 | *Capnocytophaga ochracea* | TCGTTCAGCTCAACT |
| 48 | *Capnocytophaga sputigena* | TATTACTAACAATTT |
| 49 | *Bacteroides fragilis* | CTGGTAAGTCAGTTG |
| 50 | *Bacteroides ureolyticus* | GATCTGCTGGAACCT |
| 51 | *Chlamydia pneumoniae* | TGTTAAATTTTGGGG |
| 52 | *Chlamydia trachomatis* | TGTCAAAGATCGGGG |
| 53 | *Staphylococcus aureus* | AGAGCCTTCCCCTTC |
| 54 | *Escherichia coli* | CTGATACTGGCAAGC |
| 55 | *Enterobacter cloacae* | TCAACCTGGGGAACT |
| 56 | *Enterobacter agglomerans* | GAATTTGGCAGAGAT |
| 57 | *Klebsiella pneumoniae* | GATTGGTGCCTTCGG GAACTG |
| 58 | *Klebsiella oxytoca* | CTTTGGTGCCTTCGGG AACTC |
| 59 | *Pseudomonas aeruginosa* | TGGTTCAGCAAGTTG |
| 60 | *Propionibacterium acnes* | GGAAGTGTAATCTTG |
| 61 | *Helicobacter pylori* | CTGGAGAGACTAAGC CCTCC |

### Beispiel 2:

### Identifizierung und Quantifizierung Parodontitis-assoziierter bzw. oralpathogener Pilze/Hefen mit dem erfindungsgemäßen Genchip

Die komplementären Nukleinsäuresequenzen der in Tabelle 2 aufgelisteten Referenzsequenzen werden nach der unter Beispiel 1 beschriebenen Methode auf einen Träger aufgebracht. Dabei werden vorzugsweise 15-mere verwendet. Die Referenzsequenzen werden aus den bereits geschilderten Gründen so gewählt, dass sie den spezifischen, variablen Regionen der Gene für die 28SrRNA (ribosomale RNA für die große Ribosomenuntereinheit) entsprechen. Die 28SrRNA-Gene (= 28SrDNA) werden als Target gewählt, da auch hier durch die Anwendung universeller Primer eine PCR-Reaktion zur Erhöhung der Sensitivität der Hybridisierungsreaktion vorgeschaltet werden kann und da die entsprechenden Sequenzen in den zu untersuchenden Pilzorganismen bekannt sind. Für die in Tabelle 2 aufgeführten 9 Pilzarten müssen 9 spezifische Sonden in je zwei Feldern verwendet werden, so dass sich insgesamt 18 Felder auf dem Chip ergeben. Die Feld 1.1 und Feld 1.2 enthalten zur Detektion des Pilzorganismus *Candida albicans* z.B. die Sequenz CGTCAGAGGCTATAA (komplementär zur normalen Referenzsequenz TTATAGCCTCTGACG; Tabelle 2, Zeile 1). Die Quantifizierung erfolgt durch den Einsatz der DNA bekannter Pilzmengen auf dem DNA-Chip und Korrelation der Organismenzahl zu den Signalstärken bei der Chip-Auswertung.

**Tabelle 2:**

| Liste der Nukleinsäuresequenzen der 28SrRNA-Gene der Pilzorganismen, deren komplementäre Sequenzen als Sonden auf den Genchip aufgebracht werden | | |
|---|---|---|
| **Nr.** | **Pilzspezies** | **28SrDNA-Referenzsequenz** |
| 1 | *Candida albicans* | TTATAGCCTCTGACG |
| 2 | *Candida glabrata* | CTTGGGACTCTCGCA |
| 3 | *Candida krusei* | TACACGGCCGCAGTC |
| 4 | *Candida parapsilosis* | GCGGTAGGATAAGTG |
| 5 | *Candida tropicalis* | TAGGAGAATTGCGTT |
| 6 | *Cryptococcus neoformans* | GTATTCCCTTTAGAC |
| 7 | *Aspergillus fumigatus* | CCTCGGAATGTATCA |
| 8 | *Aspergillus flavus* | CCCGGAATGTAGTGC |
| 9 | *Saccharomyces cerevisiae* | CATAGGAATGTAGCT |

### Beispiel 3:

### Identifizierung humaner Herpesviren mit dem erfindungsgemäßen Genchip

Die komplementären Nukleinsäuresequenzen der in Tabelle 3 aufgelisteten Referenzsequenzen werden mittels der bereits beschriebenen Methodik auf einen Träger aufgebracht. Dabei werden vorzugsweise 15-mere verwendet. Die Sequenzen für den Nachweis der 8 bekannten humanen Herpesviren werden so gewählt, dass sie zu den spezifischen, variablen Regionen des Gens für die virale DNA-Polymerase komplementär sind. Die DNA-Polymerase wird als Target gewählt, da auch hier durch die Anwendung universeller Primer eine PCR-Reaktion zur Erhöhung der Sensitivität der Hybridisierungsreaktion vorgeschaltet werden kann.

Für die in Tabelle 3 aufgeführten 9 Herpesvirustypen (HHV-6 wird in Typ A und Typ B unterteilt) werden 9 spezifische Sonden auf je zwei Felder gebracht, so dass sich insgesamt 18 Felder auf dem Chip ergeben. Die Feld 1.1 und Feld 1.2 enthalten z.B. zur Detektion des Herpes Simplex Virus 1 (HSV-1) die Sequenz AGGTGCGCCACTGCG (komplementär zur normalen Referenzsequenz CGCAGTGGCGCACCT; Tabelle 3, Zeile 1). Die Quantifizierung erfolgt durch den Einsatz der DNA bekannter Virusmengen auf dem DNA-Chip und die Korrelation der Virusanzahl zu den Signalstärken bei der Chip-Auswertung.

**Tabelle 3:**

| | | |
|---|---|---|
| Liste der Nukleinsäuresequenzen der humanen Herpesviren, deren komplementäre Sequenzen als Sonden auf den Genchip aufgebracht werden. | | |

| **Nr.** | **Humanes Herpesvirus** | **Referenzsequenz** |
|---|---|---|
| 1 | *Herpes Simplex Virus 1 (HSV-1)* | CGCAGTGGCGCACCT |
| 2 | *Herpes Simplex Virus 2 (HSV-2)* | CCTGGAGGCGGACCG |
| 3 | *Varizella-Zoster Virus (VZV)* | TCTGAAGAACTTCCG |
| 4 | *Zytomegalievirus (CMV)* | TGGCGAGTACCCTGT |
| 5 | *Epstein-Barr Virus (EBV)* | CGTGAGCCTGAAGGA |
| 6 | *Humanes Herpesvirus 6 (HHV-6) Typ A* | ACAGACTCACGGATA |
| 7 | *Humanes Herpesvirus 6 (HHV-6) Typ B* | ACAGACTCGCGAACA |
| 8 | *Humanes Herpesvirus 7 (HHV-7)* | CGGGGTTGTTACACA |
| 9 | *Humanes Herpesvirus 8 (HHV-8)* | TTTGTCCTCAGCGGA |

### Beispiel 4:

### Identifizierung bakterieller Resistenzgene mit dem erfindungsgemäßen Genchip.

Die komplementären Nukleinsäuresequenzen der in Tabelle 4 aufgelisteten Referenzsequenzen werden wie bereits beschrieben auf einen Träger aufgebracht. Dabei werden vorzugsweise 15-mere verwendet.

Die Referenzsequenzen werden so gewählt, dass sie den spezifischen Resistenzgen-Regionen entsprechen. Bei hoher Sequenzhomologie (z. B. *tem-*Gene) ist die Auswahl eines spezifischen Oligonukleotids für jedes Resistenzgen nicht notwendig, sondern eine Sonde kann dazu verwendet werden, mehrere (im Beispiel 2 bis 16, s. Tabelle 4) verwandte Referenzsequenzen gleichzeitig zu identifizieren. Für die ausgewählten Resistenzgene (s.o.) werden 78 spezifische Oligonukleotide auf je zwei Feldern verwendet, so dass sich insgesamt 156 Felder auf dem Chip ergeben. So enthalten z.B. die Felder 1.1 und Feld 1.2 zur Detektion des *tetA*-Gens die Sequenz CCCACACCGTTGCGG (komplementär zur normalen Referenzsequenz CCGCAACGGTGTGGG; Tabelle 3, Zeile 1).

**Tabelle 4:**

| | | |
|---|---|---|
| Liste der Nukleinsäuresequenzen der bakteriellen Resistenzgene, deren komplementäre Sequenzen auf den Genchip aufgebracht werden. | | |

| **Nr.** | **Resistenzgen** | **Referenzsequenz** |
|---|---|---|
| 1 | *tetA* | CCGCAACGGTGTGGG |
| 2 | *tetB* | CGCGCGGGCGAACGG |
| 3 | *tetC* | TTTCTAAGGCAGACC |
| 4 | *tetD* | GCGGCTGTTCAAAAA |
| 5 | *tetE* | GCAGAGGCGCAACTA |
| 6 | *tetF* | GGGGGAACATTCAAT |
| 7 | *tetG* | AGGAGACTAATCGCA |
| 8 | *tetH* | AAGCCTCTACTTTTG |
| 9 | *tetK* | CTGGAACCATGAGTG |
| 10 | *tetL* | TTTAGCGTATTAAAT |
| 11 | *tetM* | GGCAATTCTACTGAT |
| 12 | *tetO* | CCGTATACTGTTTCA |
| 13 | *tetQ* | ATCTGGAATGGAGTG |
| 14 | *tetS* | TCAGAGTACAAGTTA |
| 15 | *tetT* | GACCTTTTATCAGCG |
| 16 | *tetV* | CGACGATGTATATCC |
| 17 | *tetX* | AAAATGTAAAGCCCG |
| 18 | *tetZ* | GAGCCCAATGGCGTC |
| 19 | *tem-1, tem-6, tem-17* | TGAGCGTGGGTCTCG |
| 20 | *tem-2* | TGAGCGTGGTTCTCG |
| 21 | *tem-3, tem-10, tem-21, tem-52, tem-53, tem-54, tem-59, tem-70, tem-76, tem-77, tem-78, tem-79* | TAGAGTAAGTAGTTC |
| 22 | *tem-12, tem-20, tem-22, tem26B, tem-60* | TGAGCGTGGATCTCG |
| 23 | *tem-28, tem-29, tem-43* | AACACTGCTGCCAAC |
| 24 | *tem-47, tem-48, tem-49, tem-68, tem-72* | GGAGCCAGTAAGCGT |
| 25 | *tem-91* | TCGCCTTGATTGTTG |
| 26 | *oxa-1* | AAGATCGCATTATCA |
| 27 | *oxa-2, oxa-15* | AAGATACCTCATACA |
| 28 | *oxa-3* | CGGCCTCGACATTCA |
| 29 | *oxa-5* | CAGCTTCCACGTTCA |
| 30 | *oxa-7, oxa-10, oxa-14, oxa-16, oxa-19, oxa-28, oxa-31* | AAGATCCCCAACGCA |
| 31 | *oxa-11, oxa-17* | TACCAATGACTTAGC |
| 32 | *oxa-13, oxa-19* | TACCAATAACTTAGC |
| 33 | *oxa-15, oxa-32* | AGTTGTGGCAGACGA |
| 34 | *oxa-18* | CACGCTTGTTATCGA |
| 35 | *oxa-20* | AATCCCGGGCTCAGC |
| 36 | *oxa-22* | AGGCAAGTGCGACGA |
| 37 | *oxa-23, oxa-25, oxa-26* | TGCACGAGCAAATAA |
| 38 | *oxa-27* | AAGCCGCGCAAATAC |
| 39 | *oxa-30, oxa-33* | AAGTGTGCAACGCAA |
| 40 | *carb-2, carb-3, carb-6, pse-1, pse-5* | CAATAGCTTGCGCTA |
| 41 | *carb-4* | CAATAGCTTGTGCTA |
| 42 | *carb-5* | CACTTGCCTGTGCAA |
| 43 | *shv-1, shv-2, shv-5, shv-6, shv-7, shv-8, shv-11, shv-12, shv-13, shv-14, shv-18, shv-24, shv-25, shv-26, shv-27, shv-28* | GGCCGCACGCTGACC |
| 44 | *nimA* | CAGGCAGCATCCCGA |
| 45 | *nimB* | GGTAGAACAGGATGA |
| 46 | *nimC* | GATGCGGAACGACAA |
| 47 | *nimD* | CCTGCGGAATGACAA |
| 48 | *nimE* | TGTGGAACAAGACAA |
| 49 | *ermA* | GATCGATACTTTTTG |
| 50 | *ermB, ermAM* | AGACAGTCATCTATT |
| 51 | *ermC, ermM* | GATAAGTGAGCTATT |
| 52 | *ermD, ermK* | GGAAAACGATTCTAA |
| 53 | *ermE* | CGACCCCCGGCTGGC |
| 54 | *ermF* | TGAAAACGACACAGC |
| 55 | *ermG* | GGTAAAGAGATGTAA |
| 56 | *ermJ* | AAGTCAAAAAGCCGG |
| 57 | *ermQ* | TACAGCTATAGAACT |
| 58 | *ermT, ermGT* | TAACCGCCATTGAAA |
| 59 | *ermTR* | ACATTTTACCAAGGA |
| 60 | *mefA, mefE* | TGGTCTTGTCTATGG |
| 61 | *mphA, mphK* | CGCCACCGTCGACGA |
| 62 | *mphB* | TTTGCACAAGATAAT |
| 63 | *mphBM* | GAGTAGAATGGGTTT |
| 64 | *msrA* | GTACTTTAATTATAG |
| 65 | *ereA* | CTCACTTGTTGGCGT |
| 66 | *ereB* | GGTTCATACTTACCA |
| 67 | *linA* | GTTAGATGGTGGCTG |
| 68 | *linB* | ACGTAGCTCCGTACT |
| 69 | *mecA* | GGTTTATATCATATG |
| 70 | *vanA* | ATCGTATTAGCGTTAA AGGG |
| 71 | *vanB* | TTTTGCAGGAGGATG GCG |
| 72 | *vanC* | AACGAAATCAACACCA TGCCG |
| 73 | *vanR* | AACGACACCATTGATA ATCCG |
| 74 | *vanS* | TTTAGGGTAGAGCTTC CAGC |
| 75 | *vanH* | CTGTTTGGATTTTGAA AGGA |
| 76 | *vanY* | AACACATACTAATTCA AGGAGG |
| 77 | *vanX* | GCGTTAAAGGGGTGA TAAGC |
| 78 | *vanZ* | CACGTTAACTAGGACA TTGC |

### Beispiel 5:

### Identifizierung von Resistenz auslösenden Mutationen in bakteriellen Antibiotika-Zielmolekülen (DNA-Gyrase, Topoisomerase IV) mit dem erfindungsgemäßen Chip.

Die komplementären Nukleinsäuresequenzen der in Tabelle 5 aufgelisteten Referenzsequenzen werden in der beschriebenen Methodik als Sonden auf einen Träger aufgebracht. Dabei werden vorzugsweise 15-mere verwendet, es können jedoch auch 16- bis 25-mere zum Einsatz kommen. Die Sequenzen werden dabei so gewählt, dass die in der mutierten Referenzsequenz vorhandene Sequenzabweichung etwa in der Mitte liegt.

Die Sonden werden jeweils nur auf ein Feld aufgebracht. Bezüglich der bisher bekannten Mutationen in den *gyrA-* und parC-Genen oraler Streptokokken bzw. im *gyrB*-Gen von *T. denticola* ergeben sich 22 Felder, wobei z. B. das Feld 1.1 die Sequenz CCGTCCTTGTAGACC (komplementär zu der normalen Referenzsequenz GGTCTACAAGGACGG, Tabelle 5, Nr. 1 (Zeile 1), Spalte 3), das Feld 1.2 die entsprechende komplementäre, mutierte Referenzsequenz CCGTCCTCGTAGACC (komplementär zu der mutierten Referenzsequenz GGTCTACGAGGACGG, Tabelle 5, Nr. 1 (Zeile 1), Spalte 4; Mutation unterstrichen) trägt. Für die Belegung der Felder 2.1 und 2.2 wird die Sequenzinformation der Nr. 1 (Zeile 2) der Tabelle 5 (*gyrB*/Lys136Thr) verwendet usw.

**Tabelle 5:**

| Liste der Nukleinsäuresequenzen, deren komplementäre Sequenzen auf den Genchip aufgebracht werden (es wird nur der zentrale Bereich der 15- bis 25-mere gezeigt, die Position mit Bezug auf die Wildtyp-Referenzsequenz wird durch Angabe der Codon-Nr. spezifiziert). Zu jeder mutierten Referenzsequenz wird an definierter Stelle auf dem Träger die entsprechende komplementäre Sequenz (siehe 3. Spalte) aufgebracht. | | | | |
|---|---|---|---|---|
| **Nr.** | **Gen/Kodon-Nr.** | **normale Referenzsequenz** | **mutierte Referenzsequenz** | **Aminosäureaustausch** |
| 1 | *garB*/*136* | AAG | GAG | Lys-Glu |
| 2 | *(Treponema denticola)* | AAG | ACG | Lys-Thr |
| 3 | *gyrA*/*83* | TCT | TAT | Ser-Tyr |
| 4 | *(Streptococcus sanguis)* | TCT | TTT | Ser-Phe |
| 5 | *gyrA*/*83 (Streptococcus anginosus)* | TCC | TTC | Ser-Phe |
| 6 | *gyrA*/*87 (Streptococcus sanguis )* | GAA | AAA | Glu-Lys |
| 7 | *gyrAl87 (Streptococcus anginosus )* | GAA | CAA | Glu-Gln |
| 8 | *parC*/*79* | AGT | TTT | Ser-Phe |
| 9 | *(Streptococcus sanguis)* | AGT | ATT | Ser-Ile |
| 10 | *parC*/*79* | AGC | TTA | Ser-Leu |
| 11 | *(Streptococcus anginosus)* | AGC | ATC | Ser-Ile |

### Beispiel 6:

### Identifizierung bakterieller Gene für Virulenzfaktoren

Um Aussagen über die Aggressivität der an der individuellen Erkrankung beteiligten Bakterienstämme treffen zu können, enthält der erfindungsgemäße Genchip in dieser Ausführungsform Oligonukleotidsonden, die Gene für bestimmte Virulenzfaktoren detektieren können. Es werden die Gene für die Virulenzfaktoren Leukotoxin, Kollagenase und Fimbrillin von den Parodontitis-assoziierten Erregern *A. actinomycetemcomitans* und *P. gingivalis* für den Chip ausgewählt, da hier schon (z. T. eindeutige) Zusammenhänge mit der Virulenz der Bakterienstämme erkannt werden konnten.

Die komplementären Nukleinsäuresequenzen der in Tabelle 6 aufgelisteten Referenzsequenzen werden mittels der bereits beschriebenen Methodik auf einen Träger aufgebracht. Dabei werden vorzugsweise 15-mere verwendet, es können jedoch auch 16- bis 25-mere zum Einsatz kommen.

Die Referenzsequenzen werden so gewählt, dass sie den die Virulenzfaktoren codierenden spezifischen Genregionen entsprechen. Die dazu komplementäre Sonden werden auf je zwei Felder auf den Träger aufgebracht. Für 9 Virulenzfaktoren (s. Tabelle 6) müssen 9 spezifische Sonden verwendet werden, so dass sich insgesamt 18 Felder auf dem Chip ergeben. So enthalten die Felder 1.1 und Feld 1.2 zur Detektion des fimA (Typ I)-Gens von *Porphyromonas gingivalis* die Sequenz GATGGTGGCATTACC (komplementär zur normalen Referenzsequenz GGTAATGCCACCATC; Tabelle 6, Zeile 1 ).

**Tabelle 6:**

| Liste der Nukleinsäuresequenzen der bakteriellen Gene für Virulenzfaktoren, deren komplementäre Sequenzen auf den Genchip aufgebracht werden. | | |
|---|---|---|
| **Nr.** | **Virulenzfaktor-Gen** | **Referenzsequenz** |
| 1 | *fimA (Typ I) v. P. gingivalis* | GGTAATGCCACCATC |
| 2 | *fimA (Typ II) v. P. gingivalis* | GGTAATGCTACCATC |
| 3 | *fimA (Typ III) v. P. gingivalis* | AGTAATGCTACCATC |
| 4 | *fimA (Typ IV) v. P. gingivalis* | AGCGGAGAGGGGCGT |
| 5 | *fimA (Typ V) v. P. gingivalis* | GCTGGCTCGCCACAA |
| 6 | *prtC (Kollagenase) v. P. gingivalis* | CGCTCTGTGTATGGC |
| 7 | *Ikt Promotor v. A. actinomycetemcomitans* *(geringe Leukotoxizität)* | CTTGTCGCAAGTGCCA |
| 8 | *prtH (Cysteinproteinase) v. B. forsythus* | ACCGATCTTTGGCGCT ATCA |
| 9 | *Ikt Promotor (Deletion) v. A. actinomycetem-* *comitans (hohe Leukotoxizität)* | GCTGGATATTGTGGTT TGGT |

### Beispiel 7:

### Identifizierung von Polymorphismen (Mutationen) in Parodontitis-Risiko-assoziierten Genen.

Auf dem Genchip werden die bekannten Genpolymorphismen bzw. Allele nachgewiesen, die mit dem Parodontitis-Risiko assoziiert sind. Darüber hinaus werden Sonden eingesetzt, die Genpolymorphismen detektieren, die bisher noch nicht mit der Parodontitiserkrankung, wohl aber mit anderen Erkrankungen in Zusammenhang gebracht werden konnten. Diese werden ebenso unter den Begriff der "Parodontitis assoziierten Polymorphismen" gefaßt.

Die komplementären Nukleinsäuresequenzen der in Tabelle 7 aufgelisteten Referenzsequenzen werden - wie bereits beschrieben - auf einen Träger aufgebracht. Dabei werden vorzugsweise 15-mere verwendet, es können jedoch auch 16- bis 25-mere zum Einsatz kommen. Die Sequenzen werden dabei so gewählt, dass die in der komplementären, mutierten Referenzsequenz (hier als Allel 2 bezeichnet) vorhandene Sequenzabweichung etwa in der Mitte liegt.

Die Sonden werden jeweils nur auf ein Feld auf den Träger aufgebracht. Bezüglich der in diesem Beispiel genannten humanen Polymorphismen (s. Tabelle 7) ergeben sich 44 Felder, wobei z. B. das Feld 1.1 die Sequenz AAAGGTGCTGACCTA (komplementär zu der normalen Referenzsequenz TAGGTCAGCACCTTT; Tabelle 7, Zeile 1, Spalte 3), das Feld 1.2 die entsprechende komplementäre, mutierte Referenzsequenz AAAGGTGATGACCTA (komplementär zu der mutierten Referenzsequenz TAGGTCATCACCTTT, Tabelle 7, Zeile 1, Spalte 4; Mutation unterstrichen) trägt. Für die Belegung der Felder 2.1 und 2.2 wird die Sequenzinformation der Zeile 2 der Tabelle 7 (IL-1B (-511)) verwendet usw.

**Tabelle 7:**

| | | | |
|---|---|---|---|
| Liste der Nukleinsäuresequenzen, deren komplementäre Sequenzen auf den Genchip aufgebracht werden (es wird nur der zentrale Bereich der 15- bis 25-mere gezeigt). Zu jeder zur Referenzsequenz von Allel 1 komplementären Sequenz wird an definierter Stelle auf dem Träger die komplementäre Sequenz von Allel 2 (s. Spalte 3) aufgebracht. | | | |

| **Nr.** | **Gen (Position)** | **Referenzsequenz Allel 1** | **Referenzsequenz Allel 2** |
|---|---|---|---|
| 1 | *IL-1A (+4845)* | AGC | ATC |
| 2 | *IL-1B (-511)* | CCG | CTG |
| 3 | *IL-1B (+3954)* | TCG | TTG |
| 4 | *IL-1RA (+2018)* | CTG (korreliert mit 4 x 86bp-VNTR (Allel 1) in intron 2) | CCG (korreliert mit 2 x 86bp-VNTR (Allel 2) in intron 2) |
| 5 | *TNFα (-308)* | GGG | GAG |
| 6 | *TNFα (-238)* | CGG | CAG |
| 7 | *TNFβ (+252)* | GGT | GAT |
| 8 | *FcγRIIA (+494)* | CGT | CAT |
| 9 | *FcγRIIIA (+559)* | TGT | TTT |
| 10 | *IL-10 (-1082)* | AGG | AAG |
| 11 | *IL-10 (-819)* | ACA | ATA |
| 12 | *IL-10 (-592)* | TCC | TAC |
| 13 | *IL-4 (-590)* | TCC | TTC |
| 14 | *ET-1 (+4124)* | ATC | ACC |
| 15 | *RAGE (+1704)* | AGC | ATC |
| 16 | *NAT2 (+191)* | CGG | CAG |
| 17 | *NAT2 (+282)* | ACA | ATA |
| 18 | *NAT2 (+481)* | CTT | CCT |
| 19 | *NAT2 (+590)* | CGA | CAA |
| 20 | *NAT2 (+803)* | AAA | AGA |
| 21 | *NAT2 (+857)* | GGA | GAA |
| 22 | *VDR (+1055)* | TTG | TCG |

### Beispiel 8:

### Simultaner Nachweis von Parodontitis-assoziierten Mikroorganismen, Antibiotika Resistenzen, Virulenzfaktoren und humanen Parodontitis-assoziierten Genpolymorphismen

Wie in den Beispielen 1 bis 7 beschrieben, wird ein Genchip mit 15- bis 25-mere Oligonukleotidsonden hergestellt. Die Sonden sind dabei komplementär zu den Referenzsequenzen der Mikroorganismen (Bakterien, Pilze/Hefen, Herpesviren), der Antibiotikaresistenzgene und der Virulenzfaktoren bzw. komplementär zu den bekannten mutierten/polymorphen Abschnitten bzw. normalen Referenzsequenzen der Antibiotika-Zielmolekül-Gene sowie der Parodontitis-Risiko-assoziierten Gene. Die Anzahl der benötigten Hybridisierungsstellen für die genannten Parameter ist in Tabelle 8 dargestellt.

**Tabelle 8:**

| | | | |
|---|---|---|---|
| Parodontitis-Diagnostik mit einem erfindungsgemäßen Genchip. Es werden jeweils zwei Felder mit einer Sonde belegt. | | | |

| **Gentyp** | **Anzahl der Gene** | **Ziel des Hybridisierungs-nachweises** | **Hybridisierungstellen** |
|---|---|---|---|
| 16SrDNA (Bakterien) | 76 | Identifikation und Quantifizierung | 152 (2 x 76) |
| 28SrDNA (Pilze/Hefen) | 9 | Identifikation und Quantifizierung | 18(2x9) |
| virale DNA-Polymerase (Herpesviren) | 9 | Identifikation | 18(2x9) |
| Tetracyclin-Resistenz | 18 | Identifikation | 36(2x18) |
| β-Lactamase (β-Lactam-Antibiotika-Resistenz) | 80 | Identifikation | 50 (2 x 25) |
| 5-Nitroimidazol-Resistenz | 5 | Identifikation | 10(2x5) |
| Macrolid-Antibiotika-Resistenz | 26 | Identifikation | 40 (2 x 20) |
| Methicillin-Resistenz (*mecA*) | 1 | Identifikation | 2 (2 x 1) |
| Vancomycin-Resistenz | 9 | Identifikation | 18(2x9) |
| bakterielle DNA-Gyrase Untereinheit A (*gyrA*) | 2 | Mutationsdetektion | 10 |
| bakterielle DNA-Gyrase Untereinheit B (*gyrB*) | 1 | Mutationsdetektion | 4 |
| bakterielle Topoisomerase IV (*parC*) | 2 | Mutationsdetektion | 8 |
| Fimbrillin-Gen *fimA* (5 Typen v. *P. gingivalis*) | 5 | Identifikation | 10(2x5) |
| Kollagenase-Gen *prtC* v. *P. gingivalis* | 1 | Identifikation | 2 |
| Leukotoxin (*Ikf*)-Promotorregion v. *A. actinomy-cetemcomitans* | 2 | Identifikation | 4(2x2) |
| Cysteinproteinase-Gen *prtH* v. *B. forsythus* | 1 | Identifikation | 2 |
| Interleukin 1 A (IL-1A) | 1 | Mutationsdetektion | 2 |
| Interleukin 1 B (IL-1B) | 1 | Mutationsdetektion | 4 |
| Interleukin 1 Rezeptor | 1 | Mutationsdetektion | 2 |
| Interleukin 4 (IL-4) | 1 | Mutationsdetektion | 4 |
| Interleukin 4 Rezeptor | 1 | Mutationsdetektion | 2 |
| Interleukin 6 (IL-6) | 1 | Mutationsdetektion | 2 |
| Interleukin 1 Rezeptor Antagonist (IL-1RA) | 1 | Mutationsdetektion | 2 |
| Interleukin 10 (IL-10) | 1 | Mutationsdetektion | 6 |
| Tumornekrose- faktor α (TNFα) | 1 | Mutationsdetektion | 4 |
| Tumornekrose-faktor β (TNFβ) | 1 | Mutationsdetektion | 2 |
| FcγRIIA | 1 | Mutationsdetektion | 2 |
| FcγRIIIA | 1 | Mutationsdetektion | 2 |
| FcγRIIIB | 1 | Allelidentifikation | 4(2x2) |
| Endothelin 1 (ET-1) | 1 | Mutationsdetektion | 2 |
| Receptor for Advanced Glycation End Products (RAGE) | 1 | Mutationsdetektion | 2 |
| N-Acetyltransferase (NAT2) | 1 | Mutationsdetektion | 12 |
| Vitamin-D-Rezeptor (VDR) | 1 | Mutationsdetektion | 2 |
| HLA-A9 (3 häufigste Allele) | 1 | Allelidentifikation | 6(2x3) |
| HLA-B15 (6 häufigste Allele) | 1 | Allelidentifikation | 12(2x6) |

### Beispiel 9:

### Simultaner Nachweis von Parodontitis-assoziierten Mikroorganismen, Resistenzgenen, Virulenzfaktoren und humanen Parodontitis-assoziierten Genpolymorphismen mit einem nicht-komplementären DNA-Chip.

In diesem Beispiel werden nicht die komplementären Nukleinsäuresequenzen der in Tabelle 1-7 aufgelisteten Referenzsequenzen, sondern die Referenzsequenzen direkt als Sonden mit Hilfe von Standardtechniken auf einen geeigneten Träger aufgebracht. Analog zu den Beispielen 1 bis 8 werden in der Regel 15-mere verwendet, es können jedoch auch 16- 25-mere zum Einsatz kommen. Zu Kontrollzwecken werden die spezifischen Sonden jeweils auf zwei Felder nebeneinander auf den Träger aufgebracht; z. B. enthalten Feld 1.1 und Feld 1.2 zur Detektion des Keims *Actinobacillus actinomycetemcomitans* die Sequenz GATGTACTGACGCTG (entsprechend der normalen Referenzsequenz s. Tabelle 1, Zeile 1).

Im Falle der Mutationsdetektion werden die Sequenzen wiederum so gewählt, dass die Sequenzabweichung etwa in der Mitte der Oligonukleotidsonden liegt. Die Sonden zur Mutationsdetektion werden auch hier so auf den Träger aufgebracht, dass sich auf den einzelnen Feldern jeweils nur eine Variante der Oligonukleotide befindet, z. B. enthält Feld 2.1 die normale Referenzsequenz TAGGTCAGCACCTTT (Tabelle 7, Zeile 1, Spalte 3), das Feld 2.2 die entsprechende mutierte Referenzsequenz TAGGTCATCACCTTT (Tabelle 7, Zeile 1, Spalte 4; Mutation unterstrichen).

Durch die Wahl geeigneter Primer für die zum Nachweis der Hybridisierung notwendige Markierungsreaktion wird sichergestellt, dass die markierten Genabschnitte komplementär zu den auf dem DNA-Chip aufgebrachten Oligonukleotidsequenzen sind.

### Beispiel 10:

### Quantifizierung bakterieller Erreger mittels des erfindungsgemäßen Genchips.

Die komplementären Nukleinsäuren der in Tabelle 1 aufgeführten bakteriellen Referenzsequenzen werden in der bereits beschriebenen Methodik als Sonden auf einen Träger aufgebracht. Dabei werden vorzugsweise 15-mere verwendet. Es sind aber auch 16 - 25-mere möglich. Die Sonden belegen jeweils zwei Felder.

Die Sonden werden jeweils in großem Überschuß zu der erwartungsgemäß maximal in der Patientenprobe vorhandenen Bakterienanzahl einer Spezies (ca. 10⁷ bis 10⁸, von der nur ca. 1% (=10⁵ bis 10⁶) für die Analyse eingesetzt wird) aufgebracht, d.h. pro Feld werden ca. 10¹⁰ bis 10¹¹ identische Sondenmoleküle verwendet. Diese Mengen werden durch den Einsatz definierter Volumina von konzentrierten Oligonukleotidlösungen in einem Chip-Spotter Gerät eingestellt.

Die Quantifizierung der Keime ist unter der Annahme möglich, daß die Effizienz der PCR-Amplifikation, die mit einem fluoreszenzmarkierten Primerpaar, das die 16SrRNA aller Bakterien erkennt, durchgeführt wird, für alle Erreger entsprechend ist. Dies ist aufgrund der Ähnlichkeit der bakteriellen 16SrRNA-Gene anzunehmen und kann durch den Einsatz der DNA bekannter Bakterienmengen auf dem Chip überprüft werden. Durch die Standardisierung der Chip-Produktion und der Hybridisierungsbedingungen ist es auf diese Weise möglich, anhand der Fluoreszenzstärken die Bakterienzahl zu ermitteln.

### Anhang

**Tabelle 9:**

| | | | |
|---|---|---|---|
| Orale Mikroorganismen, die mit dem Parodontitis-Genchip erkannt werden. | | | |

| **Oralpathogener Organismus** | **Klassifikation** | **Grad der Assoziation mit Parodontaler-krankungen*** | **Erkrankungstyp/ Merkmale*** |
|---|---|---|---|
| *Actinobacillus actinomyce-temcomitans* | Bacteria | sehr hoch | bisher am besten untersucht, Leitkeim der LJP, zu ca. 40% an AP beteiligt, zahlreiche Virulenzfaktoren, antibiotische Therapie immer notwendig |
| *Porphyromonas gingivalis* | Bacteria | sehr hoch | häufig in großer Zahl bei fortgeschrittener AP, zahlreiche Virulenzfaktoren, kaum in gesundem Zahnfleisch nachweisbar |
| *Bacteroides forsythus* | Bacteria | sehr hoch | oft in sehr hoher Zahl bei refraktärer Parodontitis, häufigster Keim bei par- odontalen Erkrankungen |
| *Treponema spec. (T. pectinovorum, T. socranskii, T. vincentii, T. lecithinolyticum*) | Bacteria | sehr hoch | T. spec. können bis zu 50% der mikrobiol. Flora bei ANUG und AP repräsentieren, <1% an gesunden Stellen, zahlreiche Virulenzfaktoren, z. T. nicht kultivierbar |
| *Treponema denticola* | Bacteria | hoch | am besten charakterisierter oraler Spirochaet, Auftreten korreliert stark mit parodontalem Knochenabbau |
| *Prevotella intermedia* | Bacteria | hoch | erhöht bei ANUG und AP, wahrscheinlich mit-verantwortlich für refraktäre Formen der PA |
| *Campylobacter rectus* | Bacteria | hoch | höhere Anzahl an erkrankten Stellen mit aktiver parodontaler Zerstörung, Leukotoxinproduktion |
| *Eubacterium nodatum* | Bacteria | hoch | deutlich häufiger an PA-erkrankten Stellen |
| *Eikenella corrodens* | Bacteria | mittel | höhere Anzahl an erkrankten Stellen mit aktiver parodontaler Zerstörung, sowie bei therapie-resistenter Parodontitis, manchmal an LJP beteiligt |
| *Selenomonas noxia* | Bacteria | mutmaßlich | signifikant häufiger an PA-erkrankten Stellen |
| *Fusobacterium nucleatum* | Bacteria | mittel | häufig bei RPP |
| *Peptostreptococcus micros* | Bacteria | mittel | höhere Anzahl an erkrankten Stellen mit aktiver parodontaler Zerstörung, häufig bei schwerer generalisierter Parodontitis |
| *Prevotella nigrescens* | Bacteria | mittel | assoziiert mit Gingivitis |
| *Streptococcus intermedius* | Bacteria | mittel | häufig bei RPP, signifikant höhere Keimzahl in Nichtrauchern mit early-onset (EO) PA als in Rauchern |
| *Streptococcus mutans* | Bacteria | noch unklar | wichtiger Karieserreger |
| *Actinomyces viscosus* | Bacteria | noch unklar | wichtiger Karieserreger |
| *Campylobacter concisus* | Bacteria | mutmaßlich | signifikant höhere Keim-zahl in Rauchern mit early-onset (EO) PA als in Nichtrauchern |
| *Campylobacter gracilis* | Bacteria | mutmaßlich | signifikant höhere Keimzahl in Rauchern mit early-onset (EO) PA als in Nichtrauchern |
| *Campylobacter showae* | Bacteria | mutmaßlich | häufiger an PA-erkrankten Stellen |
| *Campylobacter sputorum* | Bacteria | mutmaßlich | häufiger an PA-erkrankten Stellen |
| *Eubacterium brachy* | Bacteria | mutmaßlich | häufiger an PA-erkrankten Stellen |
| *Eubacterium timidum* | Bacteria | mutmaßlich | häufiger an PA-erkrankten Stellen |
| *Eubacterium lentum* | Bacteria | mutmaßlich | signifikant höhere Keimzahl in Nichtrauchern mit early-onset (EO) PA als in Rauchern |
| *Eubacterium sabureum* | Bacteria | mutmaßlich | häufiger an PA-erkrankten Stellen |
| *Pseudoramibacter alactolyticus* | Bacteria | mutmaßlich | häufiger an PA-erkrankten Stellen |
| *Selenomonas sputigena* | Bacteria | mutmaßlich | signifikant höhere Keimzahl in Rauchern mit early-onset (EO) PA als in Nichtrauchern |
| *Fusobacterium periodonticum* | Bacteria | mutmaßlich | häufiger an PA-erkrankten Stellen |
| *Peptostreptococcus anaerobius* | Bacteria | mutmaßlich | häufiger an PA-erkrankten Stellen |
| *Prevotella melaninogenica* | Bacteria | noch unklar | häufiger an PA-erkrankten Stellen |
| *Wolinella succinogenes* | Bacteria | noch unklar | häufiger an PA-erkrankten Stellen |
| *Veillonella parvula* | Bacteria | noch unklar | häufiger an PA-erkrankten Stellen, beteiligt an Karies |
| *Veillonella dispar* | Bacteria | noch unklar | häufiger an PA-erkrankten Stellen, beteiligt an Karies |
| *Centipeda periodontii* | Bacteria | mutmaßlich | häufiger an PA-erkrankten Stellen |
| *Dialister pneumosintes* | | mutmaßlich | nachgewiesen in tiefen Parodontaltaschen bei AP |
| *Porphyromonas endodontalis* | Bacteria | mutmaßlich | häufiger an PA-erkrankten Stellen |
| *Streptococcus salivarius* | Bacteria | noch unklar | beteiligt an Karies |
| *Streptococcus sobrinus* | Bacteria | noch unklar | beteiligt an Karies |
| *Streptococcus sanguis* | Bacteria | noch unklar | beteiligt an Karies |
| *Streptococcus gordonii* | Bacteria | noch unklar | Karieserreger |
| *Streptococcus mitis* | Bacteria | noch unklar | beteiligt an Karies |
| *Streptococcus oralis* | Bacteria | noch unklar | evtl. beteiligt an Karies |
| *Streptococcus constellatus* | Bacteria | noch unklar | evtl. beteiligt an Karies |
| *Streptococcus anginosus* | Bacteria | noch unklar | häufiger an PA-erkrankten Stellen |
| *Lactobacillus casei* | Bacteria | noch unklar | beteiligt an Karies |
| *Lactobacillus (Olsenella) uli* | Bacteria | noch unklar | beteiligt an Karies |
| *Lactobacillus (Atopobium) rimae* | Bacteria | noch unklar | beteiligt an Karies |
| *Lactobacillus acidophilus* | Bacteria | unklar | wichtiger Karieserreger |
| *Actinomyces naeslundii* | Bacteria | mutmaßlich | Karieserreger, signifikant höhere Keimzahl in Nichtrauchern mit early-onset (EO) PA als in Rauchern, häufiger in supragingivaler als in subgingivaler Plaque bei adulter PA |
| *Actinomyces odontolyticus* | Bacteria | noch unklar | beteiligt an Karies, höhere Keimzahl bei gesundem Parodontium, häufiger in supragingivaler als in subgingivaler Plaque bei adulter PA |
| *Actinomyces israelii* | Bacteria | mutmaßlich | Karieserreger, signifikant höhere Keimzahl in Nicht-rauchern mit early-onset (EO) PA als in Rauchern, häufiger in supragingivaler als in subgingivaler Plaque bei adulter PA |
| *Actinomyces gerencseriae* | Bacteria | noch unklar | evtl. beteiligt an Karies |
| *Capnocytophaga gingivalis* | Bacteria | mutmaßlich | häufig in subgingivaler Plaque von PA-Patienten und von Diabetes-Patienten |
| *Capnocytophaga ochracea* | Bacteria | noch unklar | häufiger in supragingivaler als in subgingivaler Plaque bei adulter PA |
| *Capnocytophaga sputigena* | Bacteria | noch unklar | häufiger in supragingivaler als in subgingivaler Plaque bei adulter PA |
| *Bacteroides fragilis* | Bacteria | noch unklar | bisher keine Korrelationen bekannt |
| *Bacteroides ureolyticus* | Bacteria | noch unklar | bisher keine Korrelationen bekannt |
| *Chlamydia pneumoniae* | Bacteria | mutmaßlich | Nachweis von Chlamydia-Antigen im Taschenepithel von LJP- und AP-Patienten mit rheumatischen Erkrankungen, interessant auch wegen Beteiligung an Arthritis, Atherosklerose und Endokarditis |
| *Chlamydia trachomatis* | Bacteria | mutmaßlich | s.o. |
| *Staphylococcus aureus* | Bacteria | mutmaßlich | signifikant höhere Keimzahl in Rauchern mit early-onset (EO) PA als in Nichtrauchern |
| *Staphylococcus epidermidis* | Bacteria | noch unklar | häufig in tiefen Parodontaltaschen |
| *Escherichia coli* | Bacteria | mutmaßlich | Vorkommen bei AP, signifikant höhere Keimzahl in Rauchern mit early-onset (EO) PA als in Nichtrauchern |
| *Enterobacter cloacae* | Bacteria | mutmaßlich | Vorkommen bei AP |
| *Enterobacter agglomerans* | Bacteria | mutmaßlich | Vorkommen bei AP |
| *Klebsiella pneumoniae* | Bacteria | mutmaßlich | Vorkommen bei AP |
| *Klebsiella oxytoca* | Bacteria | mutmaßlich | Vorkommen bei AP |
| *Pseudomonas aeruginosa* | Bacteria | mutmaßlich | Vorkommen bei AP |
| *Propionibacterium acnes* | Bacteria | mutmaßlich | isoliert aus tiefen Parodontaltaschen |
| *Helicobacter pylori* | Bacteria | noch unklar | Parodontaltaschen = Reservoir für H. *pylori-*Infektion |
| *Gemella morbillorum* | Bacteria | noch unklar | Vorkommen bei AP, bisher nur wenige Untersuchungen |
| *Leptotrichia buccalis* | Bacteria | noch unklar | Vorkommen bei AP, bisher nur wenige Untersuchungen |
| *Neisseria mucosa* | Bacteria | noch unklar | Vorkommen bei AP, bisher nur wenige Untersuchungen |
| *Candida albicans* | Fungi | mutmaßlich | Karieserreger, Opportunist, signifikant höhere Keimzahl in Rauchern mit early-onset (EO) PA als in Nichtrauchern |
| *Candida glabrata* | Fungi | noch unklar | Vorkommen auch in Parodontaltaschen |
| *Candida krusei* | Fungi | noch unklar | Vorkommen auch in Parodontaltaschen |
| *Candida parapsilosis* | Fungi | noch unklar | noch keine Korrelationen bekannt |
| *Candida tropicalis* | Fungi | noch unklar | noch keine Korrelationen bekannt |
| *Cryptococcus neoformans* | Fungi | noch unklar | noch keine Korrelationen bekannt |
| *Aspergillus fumigatus* | Fungi | noch unklar | signifikant höhere Keimzahl in Rauchern mit EOP als in Nichtrauchern |
| *Aspergillus flavus* | Fungi | noch unklar | in einem Fall aus einer Parodontaltasche isoliert |
| *Saccharomyces cerevisiae* | Fungi | noch unklar | Vorkommen in Parodontaltaschen |
| *Herpes Simplex Virus 1 (HSV-1)* | Herpes-viren | hoch | positiver Nachweis in Parodontaltaschen, assoziiert mit LJP, assoziiert mit Zahnfleischentzündungen |
| *Herpes Simplex Virus 2 (HSV-2)* | Herpes-viren | noch unklar | gelegentlich mit Zahnfleischentzündungen assoziiert |
| *Varizella-Zoster Virus (VZV)* | Herpes-viren | noch unklar | Infektion kann Zahnfleischläsionen hervorrufen |
| *Zytomegalievirus (CMV)* | Herpes-viren | hoch | signifikant höhere Keimzahl an ANUG- und PA-erkrankten Stellen als an gesunden Stellen, HCMV-Aktivierung korreliert mit erhöhten Keimzahlen von A. actinomycetemcomitams bei LJP |
| *Epstein-Barr Virus (EBV)* | Herpes-viren | hoch | signifikant höhere Keimzahl an ANUG- und PA-erkrankten Stellen als an gesunden Stellen, assoziiert mit LJP |
| *Humanes Herpesvirus 6 (HHV-6) Typ A und B* | Herpes-viren | noch unklar | gelegentl. Vorkommen in parodontalen Läsionen, evtl. Beteiligung an Oralkarzinomen |
| *Humanes Herpesvirus 7 (HHV-7)* | Herpes-viren | noch unklar | Vorkommen bei Zahnfleischentzündungen |
| *Humanes Herpesvirus 8 (HHV-8)* | Herpes-viren | noch unklar | Vorkommen in parodontalen Läsionen von HIV-Patienten |

| | | | |
|---|---|---|---|
| *aktueller Wissensstand | | | |

**Tabelle 10:**

| | | |
|---|---|---|
| Antibiotika-Resistenzen, die mit dem Parodontitis-Genchip erkannt werden. | | |

| **Antibiotikaresistenz gegen** | **Resistenzmechanismus** | **Gene** |
|---|---|---|
| Tetracycline | Resistenz vorwiegend durch aktiven Efflux des Tetracyclin-Moleküls | *tet*-Gene |
| β-Lactam-Antibiotika (z. B. Penicillin, Amoxicillin...) | Spaltung des β-Lactamrings | *tem*-Gene, *oxa*-Gene, *carb*-Gene, *pse*-Gene, *shv*-Gene |
| 5-Nitroimidazol (z. B. Metronidazol) | noch unklar | *nim*-Gene |
| Macrolide (z. B. Erythromycin) und Clindamycin | Modifikation des Zielmoleküls (rRNA-Methylase, z. B. *erm*-Gene); aktiver Efflux, z. B. *mef*-Gene; Phosphorylierung des Macrolid-Moleküls, z. B. *mph-*Gene | *erm*-Gene, *mef*-Gene, *mph-Gene, msr*A-Gen, *ere*-Gene, *lin*-Gene |
| Methicillin | Affinität zu β-Lactamantibiotika, genauer Mechanismus noch unklar | *mecA* |
| Vancomycin | Synthese einer veränderten Zellwandstruktur durch: regulatorische Proteine (*vanR, vans*), Enzyme für die Synthese von veränderten Peptidoglycanen (*vanA, vanB, vanC, vanH, vanX*), akzessorische Proteine (*vanY, vanZ*) | *van*-Gene |
| Fluoroquinolone | Mutation der Zielmoleküle DNA Gyrase und Topoisomerase IV | *gyr*A, *gyr*B, *par*C |

**Tabelle 11:**

| | | | |
|---|---|---|---|
| Virulenzfaktoren oraler Mikroorganismen, die mit dem Parodontitis-Genchip erkannt werden. | | | |

| **Virulenzfaktor** | **Organismus** | **Gene** | **Charakteristika/Bes onderheiten** |
|---|---|---|---|
| Leukotoxin | *A. actinomycetemcomitans* | *Ikt* | hoch virulenter Stamm v. *A. actinomycetemcomitans*: 530bp-Deletion im Leukotoxin-Promotor bewirkt stark erhöhte Leukotoxin-Produktion; Präsenz dieses Stammes häufig bei LJP und EOP |
| Kollagenase | *P. gingivalis* | *prtC* | Korrelation der Virulenz mit dem Vorhandensein des *prtC*-Gens wird vermutet |
| Cystein-Proteinase | *B. forsythus* | *prtH* | nur bei Parodontitisassoziierten *B. forsythus*-Stämmen |
| Fimbrillin | *P. gingivalis* | *fimA* Typ I-V | Typ des *fim*A-Gens beeinflusst die Eigenschaften der Fimbrien; Einfluss auf die Virulenz wird vermutet |

**Tabelle 12:**

| | | |
|---|---|---|
| Humane Gene, die mit dem Parodontitis-Risiko assoziiert sind bzw. assoziiert sein könnten. | | |

| **Gen(e)** | **Funktion des Genprodukts** | **Korrelationen von Genpolymorphismen/spez. Allelen mit parodontalen Erkrankungen*** |
|---|---|---|
| Interleukin 1A | entzündungsförderndes Zytokin | +4845 Genpolymorphismus: Allel 2 korreliert mit schwerem Verlauf der adulten Parodontitis |
| Interleukin 1B | entzündungsförderndes Zytokin | + 3954 Genpolymorphismus: Allel 2 korreliert mit schwerem Verlauf der adulten Parodontitis |
| Interleukin 1 Rezeptor | Membranrezeptor für IL-1 | noch nicht bekannt |
| Interleukin 4 | Zytokin, das die humorale Immunantwort übermittelt | 2 Genpolymorphismen korrelieren mit dem Auftreten der EOP |
| Interleukin 4 Rezeptor | Membranrezeptor für IL-4 | noch nicht bekannt |
| Interleukin 6 | entzündungsförderndes Zytokin | noch nicht bekannt |
| Interleukin 1 Rezeptor Antagonist | anti-inflammatorisches Molekül | Pos. 8006 (Genebank Acc. No. X64532) Genpolymorphismus: Allel 2 (korreliert mit Allel 2 eines 86-bp-VNTRs in Intron 2) ist mit vielen inflammatorischen Erkrankungen assoziiert, u. a. mit früh beginnender Parodontitis |
| Tumornekrosefaktor α | entzündungsförderndes Zytokin, übermittelt das Signal zum Knochenabbau als Reaktion auf bakterielle Endotoxine | -308 Genpolymorphismus: Allel 1 ist häufiger bei Patienten mit fortgeschrittener Parodontitis |
| Tumornekrosefaktor β | entzündungsförderndes Zytokin | noch unklar |
| Fcγ RII (CD32) Rezeptor | Rezeptor für die konstante Domäne (FcR) der Immunglobuline vom Typ G | Fcγ RIIa-R/R131 Genotyp spielt vermutlich eine Rolle bei der Pathogenese der LJP und EOP |
| Fcγ RIII (CD16) Rezeptor | Rezeptor für die konstante Domäne (FcR) der Immunglobuline vom Typ G | Fcγ Rllla 158F-Allel korreliert mit dem Wiederauftreten der adulten Parodontitis Fcγ Rlllb NA2-Allel (4-Aminosäuren-Austausch) stellt einen Risikofaktor für die refraktäre adulte Parodontitis dar |
| Interleukin 10 | entzündungshemmendes Zytokin | noch unklar |
| Endothelin 1 | vasoaktives mitogenes Peptid | +4124-Genpolymorphismus (+Allel) erhöht wahrscheinlich das Parodontitis-Risiko, wenn er zusammen mit TNF-β-Genpolymorphismus auftritt |
| Receptor for Advanced Glycation End Products | entzündungsfördernder Signal-transduktions-rezeptor | +1704-Genpolymorphismus: T-Allel korreliert mit einer verringerten Neigung zu chronischer Parodontitis |
| N-Acetyltransferase | Entgiftungsenzym, anhand von mehreren Genpolymorphismen Unterscheidung zwischen "langsamen Acetylierern" (Entgiftern) und "schnellen Acetylierern" | Langsame Acetylierer (mindestens 2 mutierte Allele) haben ein höheres Parodontitis-Risiko, v. a. wenn sie Raucher sind |
| Vitamin-D-Rezeptor | wichtiger Regulator des Knochenstoffwechsels | +1055-Genpolymorphismus: T-Allel korreliert mit signifikanter Erhöhung des Risikos für EOP |
| HLA-Antigene | Zellerkennung, beteiligt an der individuellen Immunantwort | Allele HLA-A9 und HLA-B15 korrelieren mit generalisierter, früh beginnender Parodontitis |

| | | |
|---|---|---|
| *aktueller Wissensstand | | |

## Patentansprüche

1. Verfahren zur Herstellung eines Genchips zur Untersuchung oraler Krankheitsfaktoren umfassend das Aufbringen von Oligonukleotiden oder deren Komplementärsträngen auf einen Träger, wobei die Oligonukleotide Sonden für mindestens einen der in Tabelle 1 genannten Parodontitis assoziierten Mikroorganismen und mindestens einen der in Tabelle 7 genannten Parodontitis assoziierten humanen Genpolymorphismen umfassen.

2. Verfahren nach Anspruch 1, **dadurch gekennzeichnet, daß** zusätzlich Oligonukleotide oder deren Komplementärstränge auf den Träger aufgebracht werden, die Sonden für Virulenzfaktoren oralpathogener Bakterien darstellen.

3. Verfahren nach Anspruch 2, **dadurch gekennzeichnet, daß** die Sonden für die Virulenzfaktoren mit Nukleotidsequenzen der Tabelle 6 oder mit dazu komplementären Oligonukleotiden hybridisieren.

4. Verfahren zur Untersuchung oraler Krankheitsfaktoren, **dadurch gekennzeichnet, daß** Oligonukleotide aus einer Patientenprobe oder aus einer Patientenprobe synthetisierte Oligonukleotide mit Oligonukleotidsonden oder deren Komplementärsträngen hybridisiert werden, wobei die Oligonukleotidsonden auf einem Träger aufgebracht werden, und Sonden für mindestens einen der in Tabelle 1 genannten Parodontitis assoziierten Mikroorganismen und mindestens einen der in Tabelle 7 genannten Parodontitis assoziierten humanen Genpolymorphismen umfassen, und die Hybridisierung anschließend nachgewiesen wird.

5. Träger mit Oligonukleotiden zur Untersuchung oraler Krankheitsfaktoren, **dadurch gekennzeichnet, daß** die Oligonukleotide Sonden sowohl für mindestens einen der in Tabelle 1 genannten Parodontitis assoziierten Mikroorganismen als auch mindestens einen der in Tabelle 7 genannten Sonden für Parodontitis assoziierten humanen Genpolymorphismen umfassen.

## Claims

1. Method for the production of a gene chip for analysis of oral disease factors comprising the application of oligonucleotides or their complementary strands on a carrier, whereby the oligonucleotides comprise probes for at least one of the periodontitis-associated microorganisms as listed in table 1 and at least one of the periodontitis-associated human gene polymorphisms as listed in table 7.

2. Method according claim 1, **characterised in that** additional oligonucleotides or their complementary strands which represent probes for virulence factors of orally pathogenic bacteria are applied on the carrier.

3. Method according claim 2, **characterised in that** the probes for the virulence factors hybridise with the nucleotide sequences of table 6 or with the respective oligonucleotides complementary thereto.

4. Method for analysis of oral disease factors, **characterised in that** oligonucleotides from a patient sample or oligonucleotides synthesised from a patient sample are hybridised with oligonucleotides probes or their complementary strands, whereby the oligonucleotide probes which comprise a probe for at least one of the periodontitis-associated microorganisms as listed in table 1 and at least one of the periodontitis-associated human gene polymorphisms as listed in table 7, are applied on a carrier and the hybridisation will be detected afterwards.

5. Carrier with oligonucleotides for analysis of oral disease factors, **characterised in that** the oligonucleotides comprise probes for both at least one of the periodontitis-associated microorganisms as listed in table 1 and at least one of the periodontitis-associated human gene polymorphisms as listed in table 7.

## Revendications

1. Procédé de fabrication d'une puce génétique destinée à examiner des facteurs de maladies orales, comprenant l'application d'oligonucléotides ou de leurs brins complémentaires sur un support, dans lequel les oligonucléotides comprennent des sondes pour au moins un des microorganismes associés à la parodontite et cités dans le tableau 1 et pour au moins un des polymorphismes génétiques humains associés à la parodontite et cités dans le tableau 7.

2. Procédé selon la revendication 1, **caractérisé en ce que** sont en outre appliqués sur le support des oligonucléotides ou leurs brins complémentaires qui représentent des sondes pour les facteurs de virulence de bactéries pathogènes orales.

3. Procédé selon la revendication 2, **caractérisé en ce que** les sondes pour les facteurs de virulence s'hybrident avec des séquences de nucléotides du tableau 6 ou avec des oligonucléotides qui leur sont complémentaires.

4. Procédé d'examen de facteurs de maladies buccales, **caractérisé en ce que** des oligonucléotides issus d'un échantillon de patient ou des oligonucléotides synthétisés à partir d'un échantillon de patient sont hybridés avec des sondes oligonucléotidiques ou leurs brins complémentaires, les sondes oligonucléotidiques étant appliquées ou disposées sur un support, et comportant des sondes pour au moins un des microorganismes associés à une parodontite et cités au tableau 1 et pour au moins un des polymorphismes génétiques humains associés à la parodontite et cités au tableau 7, et l'hybridation étant ensuite détectée.

5. Support comportant des oligonucléotides permettant d'examiner des facteurs de maladies buccales, **caractérisé en ce que** les oligonucléotides comportent des sondes tant pour au moins un des microorganismes associés à une parodontite et cités au tableau 1 que pour au moins un des polymorphismes génétiques humains associés à une parodontite et cités au tableau 7.
